# EUROPÄISCHE PATENTSCHRIFT

(11) **EP 0 591 798 B1**
(45) Veröffentlichungstag und Bekanntmachung des Hinweises auf die Patenterteilung: **28.08.1996**
(21) Anmeldenummer: 93115521.2
(22) Anmeldetag: 25.09.1993
(51) Int. Cl.: C07C 239/20

(54) **Verfahren zur Herstellung von O-substituierten Hydroxylammoniumsalzen**
Process for preparing O-substituted hydroxylammonium salts
Procédé pour la préparation de sels d'hydroxylammonium O-subsitué

(30) Priorität: 05.10.1992 DE 4233333
(43) Veröffentlichungstag der Anmeldung: 13.04.1994
(73) Patentinhaber: BASF Aktiengesellschaft, 67063 Ludwigshafen (DE)
(72) Erfinder: Klein, Ulrich, Dr., D-6703 Limburgerhof (DE); Buschmann, Ernst, Dr., D-6700 Ludwigshafen (DE); Keil, Michael, Dr., D-6713 Freinsheim (DE); Goetz, Norbert, Dr., D-6520 Worms 1 (DE); Hartmann, Horst, D-6737 Boehl-Iggelheim (DE)

(56) Entgegenhaltungen:
- EP-A- 0 259 850
- CHEMICAL ABSTRACTS, Band 96, Nr. 17, 26. April 1982, Columbus, Ohio, USA; A.D. ZORINA et al, "Method for the synthesis O-carboxy-methylhydroxylamine hydrochloride", Seite 696, Zusammenfassung Nr. 142 207d

## Beschreibung

Die vorliegende Erfindung betrifft ein neues Verfahren zur Herstellung von O-substituierten Hydroxylammoniumsalzen der allgemeinen Formel 1

R-O-NH₂·HX (1)

in der R Alkyl mit 1 bis zu 5 C-Atomen insbesondere Methyl, Ethyl, Propyl, 1-Methylethyl, Butyl, 1-Methylpropyl, 1,1-Dimethylpropyl, Pentyl, 1-Methylbutyl, 2-Methylbutyl, 3-Methylbutyl, 1-Ethylpropyl, 2-Ethylpropyl und X Halogen oder Hydrogensulfat bedeuten.

O-substituierte Hydroxylamine sind bekannt. Sie sind wichtige Zwischenprodukte für die Herstellung von Pflanzenschutzmitteln, Arzneimitteln oder Feinchemikalien.

Aus der Literatur sind Methoden zur Synthese von O-substituierten Hydroxylaminen bekannt. In Houben Weyl, Methoden der organischen Chemie, Bd. 10,1, S. 1186 wird ein Überblick über diese Methoden gegeben.

O-substituierte Hydroxylamine lassen sich durch Hydrolyse von O-substituierten Benzaldehydoximen (Petraczek, et. al, Ber. dtsch. Chem. Ges. 16, 823 (1883)), O-substituierten Hydroximsäurealkylestern (Werner et. al., Ber. dtsch, Chem. Ges. 26, 1567 (1983), Ber. dtsch, Chem. Ges. 27, 3350 (1894), US 4965390), O-substituierten Benzophenoximen (Semper et. al., Ber. dtsch. Chem. Ges. 51, 928 (1918)), O-substituierten Hydroxamsäuren (EP 306 936), US 5 008 455) und O-substituierten N-Hydroxyurethanen (Winternitz et. al., Bull, Soc. chem. Fr. [5], 1958, 664, DE 32 45 503) mit Mineralsäure herstellen.

Eine weitere Methode stellt die Umsetzung von Hydroxylamin-O-sulfonsäure mit Alkoholen dar (EP 341 693).

Alle diese Methoden sind für die großtechnische Anwendung nicht geeignet, weil die Ausbeuten niedrig sind und die Hydrolysen bei hohen Temperaturen durchgeführt werden müssen, was bei den eingesetzten und entstehenden Verbindungen sicherheitstechnisch bedenklich ist, weil diese labile und energiereiche N-O-Bindungen enthalten, die sich heftig zersetzen können.

Die für die Herstellung nach den beschriebenen Verfahren benötigten Vorprodukte müssen vielfach aufwendig hergestellt werden und sind oft mit N-substituierten Produkten verunreinigt. Dies führt bei der Hydrolyse zu Gemischen von N- und O-monosubstituierten und N-O-disubstituierten Hydroxylaminen.

Ein wirtschaftliches und sicherheitstechnisch etwas besser zu beherrschendes Verfahren für die großtechnische Herstellung von O-substituierten Hydroxylaminen ist deshalb die bekannte Hydrolyse von O-substituierten Acetonoximen.

Die als Vorstufen benötigten Acetonoximether lassen sich in guten Ausbeuten ohne Verunreinigung durch N-alkylierte Produkte herstellen. Die Acetonoximderivate sind ziemlich stabil.

Acetonoximether lassen sich mit Salzsäure durch Erhitzen unter Rückfluß hydrolysieren. So stellen Bernhard et. al. (Liebigs Am. Chem. 257, 203 (1890)) Benzyloxyamin-Hydrochlorid mit 50 % Ausbeute her. Borek et. al. (J. Am. Chem. Soc. 58, 2020 (1936)) synthetisieren Carboxymethylenoxyamin-Hydrochlorid in 50 % Ausbeute und Holland et. al. (J. Chem. Soc. 1948, 182) erhalten Diethylaminoethylenoxyamin nach dieser Methode. Eine Ausbeute des isolierten Produkts wird nicht angegeben. Mit 47 % Ausbeute stellen Brossi et. al. (Heterocycles 20, 839 (1983)) 3'-(2,4,5-Trichlorphenoxy)propyloxyamin-Hydrochlorid durch Hydrolyse in ethanolischer Salzsäure her. Bei den bekannten Verfahren fallen die End-Produkte in unsauberem Zustand an und müssen durch Umkristallisation gereinigt werden. Die Ausbeuten sind für eine industrielle Anwendung zu niedrig.

Die Hydrolyse von Acetonoximethern zu O-substituierten Hydroxylaminen und Aceton stellt eine Gleichgewichtsreaktion dar, deren Gleichgewicht auf der Seite der Oximether liegt.

Eine Gleichgewichtsverschiebung zu den gewünschten O-substituierten Hydroxylaminen kann in üblicher Weise durch Entfernen eines der in der Mischung im Gleichgewicht vorhandenen Produkte aus dieser Mischung bewerkstelligt werden. Ökonomisch vorzuziehen ist das Abdestillieren von Aceton aus der Mischung.

So wird z. B. in Org. Synth., Coll. Vol. 3, S. 172 die Hydrolyse von Acetonoxim-O-(Carboxymethylen)ether mit wässriger Salzsäure unter gleichzeitigem Abdestillieren von Aceton beschrieben. Das Verfahren ergibt nur 66 - 72 % Ausbeute und läßt sich nicht auf die Herstellung der erfindungsgemäß herzustellenden Verbindungen übertragen.

Hydrolysiert man nämlich Acetonoximether, die Substituenten mit niederem Molekulargewicht tragen, mit wässrigen Mineralsäuren und verschiebt das Gleichgewicht durch Abdestillieren von Aceton, so destilliert gleichzeitig mit dem Aceton auch der Acetonoximether über.

Man muß daher einen großen Überschuß an Acetonoximether für die Umsetzung verwenden. Tritt während der Destillation eine Zersetzung des Hydroxylaminsalzes auf, dann findet sich im Rückstand der Destillation ein Ammoniumsalz z.B. Ammoniumchlorid.

In DE-A 36 31 071 (entspricht EP-A 259 850) wird ein Verfahren zur Hydrolyse von Acetonoximethern mit Salzsäure beschrieben, das auch für Acetonoximether, die Substituenten mit einem niederen Molekulargewicht besitzen, geeignet ist. Hierzu ist jedoch eine komplizierte Spezialapparatur erforderlich. Die Reaktionstemperatur von 70 - 140°C ist sicherheitstechnisch bedenklich, weil sie in der Nähe der Zersetzungstemperatur der O-substituierten Hydroxylamine liegt, die etwa 140°C beträgt.

Es besteht daher Bedarf an einem diskontinuierlichen Verfahren, das in einer Standard-Apparatur durchgeführt werden kann und das für Acetonoximether, die Substituenten mit einem niedrigen Molekulargewicht tragen, geeignet ist.

Es wurde nun überraschend gefunden, daß es durch Zusatz von unter den Reaktionsbedingungen inerten Stoffen aus der Gruppe: aliphatische C₅-C₁₂-Kohlenwasserstoffe, cycloaliphatische C₅-C₁₂-Kohlenwasserstoffe, unsubstituierte oder substituierte aromatische Kohlenwasserstoffe gelingt, das bei der Umsetzung entstehende Aceton aus dem Reaktionsgemisch abzudestillieren, ohne zusammen mit dem Aceton auch den Acetonoximether abzudestillieren.

Solche aliphatischen C₅-C₁₂-Kohlenwasserstoffe sind beispielsweise Pentan, Hexan, Heptan, Isopentan, Isohexan, Isoheptan; cycloaliphatische C₅-C₁₂-Kohlenwasserstoffe sind beispielsweise Cyclopentan, Cyclohexan, Cycloheptan; die aromatischen Kohlenwasserstoffe sind beispielsweise Alkylbenzol, z.B. Toluol, Benzol, o-, m- und p-Xylol, Halogenbenzol, wie Chlorbenzol, o-, m- und p-Dichlorbenzol, Alkoxybenzol, Petrolether, Ligroin.

Gemäß dem erfindungsgemäßen Verfahren werden Acetonoximether, die Substituenten mit einem niedrigen Molekulargewicht besitzen, nicht aus dem Reaktionsgemisch abdestilliert, sondern man destilliert eine Mischung von Aceton, Zusatzstoff, evtl. Säure und Wasser ab.

Daher ist es jetzt möglich, die Hydrolyse von Acetonoximether ohne Verlust an Acetonoximether durchzuführen. Dies erbringt eine wesentlich höhere Ausbeute und Reinheit der Endprodukte.

Die übliche Vorgehensweise sieht wie folgt aus: Man legt den Acetonoximether, einen Überschuß an wässriger Mineralsäure und den inerten Zusatzstoff vor, destilliert anschließend ein Gemisch aus Aceton, inertem Zusatzstoff, evtl. Säure und Wasser so lange ab, bis kein Aceton mehr überdestilliert und isoliert das O-substituierte Hydroxylamin als Salz oder gegebenenfalls als freie Base. Eine Reinigung, z. B. durch Umkristallisation ist nicht mehr erforderlich.

Die Reaktionstemperatur liegt zwischen O und 100°C, bevorzugt zwischen 40 und 80°C. Das erfindungsgemäße Verfahren ist sicherheitstechnisch unbedenklich, weil ein ausreichender Temperatur-Abstand zu den Zersetzungstemperaturen der O-substituierten Hydroxylamine eingehalten wird.

Die Mineralsäuren, z.B. Salzsäure, Bromwasserstoffsäure, Jodwasserstoffsäure, Schwefelsäure werden im Allgemeinen mindestens in molaren Mengen eingesetzt. Üblicherweise verwendet man sie in einem Überschuß von 10 bis 500 %, vorzugsweise in einem Überschuß von 30 bis 100 %, bezogen auf den Acetonoximether.

Der Reaktionspartner Wasser wird im Allgemeinen in einem bis zu 100fachen molaren Überschuß bezogen auf den Acetonoximether eingesetzt, da er auch als Lösungsmittel wichtig ist. Üblicherweise verwendet man einen 5 - 100fachen molaren Überschuß bezogen auf den Acetonoximether, bevorzugt einen 5 - 10fachen molaren Überschuß.

Die O-substituierten Hydroxylamine können in Form ihrer Salze oder nach Zusatz von Alkalien als freie Basen isoliert werden.

Will man die Salze isolieren, so entfernt man beispielsweise das überschüssige Wasser durch azeotropes Abdestillieren mit Hilfe des inerten Zusatzstoffes. Man erhält dann eine Suspension des Salzes in dem inerten Zusatzstoff und kann das Salz durch Filtration aus der Suspension abtrennen.

Will man die O-substituierten Hydroxylamine als freie Base isolieren, so stellt man nach beendeter Reaktion einen pH-Wert von etwa 10 ein und isoliert die flüchtigen O-substituierten Hydroxylamine durch Destillation. Schwerflüchtige O-substituierte Hydroxylamine isoliert man durch Extraktion und anschließendes Eindampfen des Extraktes unter vermindertem Druck.

Unter technischen Gesichtspunkten, z. B. zur Erhöhung der Raum/Zeit-Ausbeute, kann es zweckmäßig sein, daß man die Reaktion abbricht, bevor ein vollständiger Umsatz erreicht wurde. In diesem Fall empfiehlt es sich, die Verfahrens-Variante mit der Isolierung der O-substituierten Hydroxylammoniumsalze anstelle der Hydroxylamine durchzuführen, weil sich dann der unumgesetzte Acetonoximether nach der Abtrennung des Hydroxylammoniumsalzes im Filtrat zusammen mit dem inerten Zusatzstoff befindet. Das Filtrat kann für die nächste Umsetzung wiederverwendet werden.

### Vorschrift 1

### O-Methylhydroxylamin-Hydrochlorid, Hydrolyse ohne Zusatzstoff (nicht erfindungsgemäß)

174 g (2 Mol) Acetonoxim-O-methylether und 296 g (3 Mol) konzentrierte Salzsäure werden in einem Glaskolben auf 100°C erhitzt. Über eine Kolonne mit 30 cm Länge und 2,5 cm Durchmesser, die mit 5 mm Glasringen gefüllt ist, destilliert man bei einem Rücklauf zu Ablauf-Verhältnis von 6 : 1 solange bis kein Aceton mehr abdestilliert.

Die Kolbeninnentemperatur liegt zwischen 100 und 110°C. Insgesamt destilliert man 132 g einer Mischung aus 66,4 % Aceton, 14 % Wasser und 14,8 % Acetonoxim-O-methylether ab. Es gelangen 15 % des eingesetzten Acetonoxim-O-methylethers in das Destillat. Anschließend wird der Kolbeninhalt durch Eindampfen unter vermindertem Druck zur Trockene gebracht.

Ausbeute: 57 %, 112 g 85 %iges Produkt

Laut ¹H-NMR-Spektrum enthält das Produkt 15 % Ammoniumchlorid.

Schmelzpunkt: 116 - 120°C

### Vorschrift 2

### O-Methylhydroxylamin-Hydrochlorid, Hydrolyse ohne Zusatzstoff (nicht erfindungsgemäß)

Die Synthese wird wie in Vorschrift 1 beschrieben durchgeführt, jedoch destilliert man im Vakuum von 250 - 150 mbar, so daß die Kolbeninnentemperatur bei 75°C liegt. In das Destillat gelangen 30 % des eingesetzten Acetonoxim-O-methylethers.

Ausbeute: 70 %, 119 g

Laut ¹H-NMR-Spektrum enthält das Produkt kein Ammoniumchlorid.

Schmelzpunkt: 149 - 151°C

### Beispiel 1

### Herstellung von O-Methylhydroxylamin-Hydrochlorid, Hydrolyse mit Zusatzstoff (erfindungsgemäß)

750 ml Cyclohexan, 174 g (2 Mol) Acetonoxim-O-methylether, 296 g (3 Mol) konzentrierte Salzsäure werden auf 75°C erhitzt. Unter den in Beispiel 1 beschriebenen Destillationsbedingungen destilliert man bei einer Kolbeninnentemperatur von 75 - 78°C und einer Übergangstemperatur von 52 - 72°C ein zweiphasiges Destillat ab:

### Obere Phase:

750 g, bestehend aus 11,0 % Aceton und 89,0 % Cyclohexan

### Untere Phase:

119 g, bestehend aus 31,3 % Aceton, 8,1 % Cyclohexan, 53,0 % Wasser und 7,7 % Chlorwasserstoff.

Während der Destillation werden weitere 900 ml Cyclohexan zugegeben. Anschließend destilliert man das Wasser mit dem Cyclohexan azeotrop ab, und erhält als wässriges Destillat 100 g, bestehend aus 3,6 % Cyclohexan, 77,4 % Wasser und 17,6 % Chlorwasserstoff. Im Destillationsrückstand wird das ausgefallene O-Methylhydroxylamin-Hydrochlorid von Cyclohexan abgesaugt und getrocknet.

Ausbeute: 90 %, 150 g Produkt
Schmelzpunkt: 148 - 150°C

| Elementaranalyse: | | | | | |
|---|---|---|---|---|---|
| Ber. | C. 14,38, | H 7,24, | O 19,16, | N 16,77, | Cl 42,45 % |
| GGF. | C 14,4, | H 7,3, | O 19,2, | N 17,0, | Cl 42,3 % |

### Beispiel 2

### Herstellung von O-Ethylhydroxylamin-Hydrochlorid (erfindungsgemäß)

202 g (2 Mol) Acetonoxim-O-ethylether werden wie in Beispiel 1 für Acetonoxim-O-methylether beschrieben umgesetzt und aufgearbeitet.

Ausbeute: 89 %, 173 g Produkt
Schmelzpunkt: 110 - 113°C

### Beispiel 3

### Herstellung von O-Methylhydroxylamin-Hydrochlorid, Hydrolyse mit Zusatzstoff (erfindungsgemäß)

174 g (2 Mol) Acetonoxim-O-methylether, 296 g (3 Mol) konzentrierte Salzsäure und 593 g Hexan werden auf 65°C erhitzt. Unter den in Vorschrift 1 beschriebenen Destillationsbedingungen destilliert man bei einer Kolbeninnentemperatur von 64 - 65°C und einer Übergangstemperatur von 48 - 58°C ein Gemisch von Aceton, Hexan und Wasser ab bis kein Aceton mehr übergeht. Anschließend wird mit Hilfe von Hexan das restliche Wasser azeotrop abdestilliert. Das im Destillationsrückstand ausgefallene O-Methylhydroxylamin-hydrochlorid wird von Hexan abgesaugt und getrocknet.

Ausbeute: 80 %, 113 g Produkt
Schmelzpunkt: 148 - 151°C

## Patentansprüche

1. Verfahren zur Herstellung von O-substituierten Hydroxylammoniumsalzen der allgemeinen Formel 1,
R-O-NH₂·HX (1)
in der R eine C₁-C₅-Alkylgruppe und X Halogen oder Hydrogen-sulfat bedeutet, durch Umsetzung der entsprechenden Acetonoximether der allgemeinen Formel 2 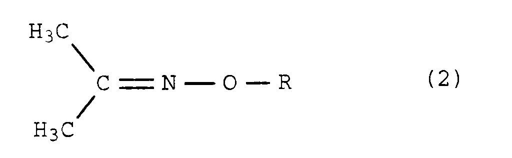 mit Wasser und einer Mineralsäure HX, dadurch gekennzeichnet, daß man den Ausgangsverbindungen einen inerten Stoff aus der Gruppe: aliphatische C₅-C₁₂-Kohlenwasserstoffe, cycloaliphatische C₅-C₁₂-Kohlenwasserstoffe, unsubstituierte oder substituierte aromatische Kohlenwasserstoffe zusetzt und das bei der Umsetzung entstehende Aceton bei Reaktionstemperaturen zwischen 0°C und 100°C abdestilliert, ohne zusammen mit dem Aceton den Acetonoximether abzudestillieren.

2. Verfahren nach Anspruch 1, dadurch gekennzeichnet, daß man die Umsetzung in einem Temperaturbereich von 40 - 80°C durchführt und das Aceton gegebenenfalls im Vakuum abdestilliert.

3. Verfahren nach Anspruch 1, dadurch gekennzeichnet, daß man Wasser in einer Menge von einem 5- bis 100-fachen molaren Überschuß bezogen auf den Acetonoximether verwendet.

4. Verfahren nach Anspruch 1, dadurch gekennzeichnet, daß man die Mineralsäure HX in einer Menge von einem 30 - 100%-igen molaren Überschuß bezogen auf den Acetonoximether verwendet.

5. Verfahren nach Anspruch 1, dadurch gekennzeichnet, daß man nach dem Ende der Acetonabtrennung die Säure HX und Wasser zusammen mit dem Inertstoff azeotrop abdestilliert und das zurückbleibende O-substituierte Hydroxylamin als HX-Salz erhält.

6. Verfahren zur Herstellung von O-Methyl- oder O-Ethylhydroxylammoniumsalzen gemäß Anspruch 5, dadurch gekennzeichnet, daß man als Acetonoximether der allgemeinen Formel 2 Acetonoxim-o-methylether oder Acetonoxim-O-ethylether einsetzt.

## Claims

1. A process for preparing O-substituted hydroxylammonium salts of the formula 1
R-O-NH₂·HX (1)
where R is C₁-C₅-alkyl and X is halogen or hydrogen sulfate, by reacting the corresponding acetone oxime ether of the formula 2 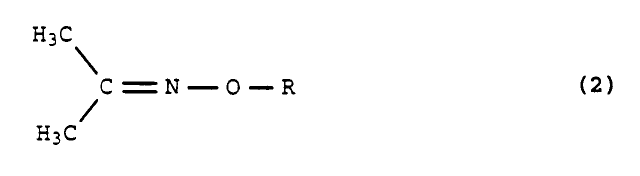 with water and a mineral acid HX, wherein an inert substance from the group consisting of aliphatic C₅-C₁₂-hydrocarbons, cycloaliphatic C₅-C₁₂-hydrocarbons and unsubstituted or substituted aromatic hydrocarbons is added to the starting compounds, and the acetone produced in the reaction as from 0 to 100°C is removed by distillation without distilling out the acetone oxime ether together with the acetone.

2. A process as claimed in claim 1, wherein the reaction is carried out at 40-80°C, and the acetone is removed by distillation under reduced pressure where appropriate.

3. A process as claimed in claim 1, wherein water is used in a 5-100-fold molar excess based on the acetone oxime ether.

4. A process as claimed in claim 1, wherein the mineral acid HX is used in a 30-100 % molar excess based on the acetone oxime ether.

5. A process as claimed in claim 1, wherein, after completion of the acetone removal, the acid HX and water are removed together with the inert substance by azeotropic distillation, and the remaining O-substituted hydroxylamine is obtained as HX salt.

6. A process for preparing O-methyl- or O-ethylhydroxylammonium salts as claimed in claim 5, wherein acetone oxime O-methyl ether or acetone oxime O-ethyl ether is used as acetone oxime ether of the formula 2.

## Revendications

1. Procédé de préparation de sels d'hydroxylammonium substitués sur l'oxygène, répondant à la formule générale 1
R-O-NH₂·HX (1)
dans laquelle R représente un groupe alkyle en C1-C5 et X un halogène ou un radical bisulfate, par réaction des éthers d'acétonoximes de formule générale 2 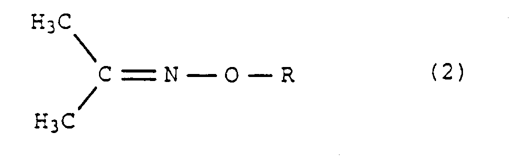 avec l'eau et un acide minéral HX, caractérisé par le fait que l'on ajoute aux composés de départ une substance inerte choisie dans le groupe formé par les hydrocarbures aliphatiques en C5-C12, les hydrocarbures cycloaliphatiques en C5-C12, les hydrocarbures aromatiques non substitués ou substitués et on distille l'acétone formée dans la réaction à des températures de réaction de 0 à 100°C, sans distiller l'éther d'acétonoxime avec l'acétone.

2. Procédé selon la revendication 1, caractérisé par le fait que l'on effectue la réaction dans l'intervalle de température de 40 à 80°C et on distille l'acétone le cas échéant sous vide.

3. Procédé selon la revendication 1, caractérisé par le fait que l'on utilise l'eau à un excès molaire de 5 à 100 fois par rapport à l'éther d'acétonoxime.

4. Procédé selon la revendication 1, caractérisé par le fait que l'on utilise l'acide minéral HX en quantité correspondant à un excès molaire de 30 à 100 % par rapport à l'éther d'acétonoxime.

5. Procédé selon la revendication 1, caractérisé par le fait que, après la séparation de l'acétone, on soumet l'acide HX et l'eau à distillation azéotropique avec la substance inerte et on recueille l'hydroxylamine substituée à l'oxygène en résidu à l'état de sel de HX.

6. Procédé de préparation des sels d'O-méthyl- ou d'O-éthylhydroxylammonium selon la revendication 5, caractérisé par le fait que l'on met en oeuvre l'éther O-méthylique ou l'éther O-éthylique de l'acétonoxime.
